# EUROPEAN PATENT APPLICATION

(11) **EP 4 691 427 A1**
(43) Date of publication of application: **11.02.2026**
(21) Application number: 23929866.4
(22) Date of filing: 28.09.2023
(51) Int. Cl.: A61F 2/82, A61F 2/86, A61F 2/95, A61F 2/958, A61B 17/00

(54) **SEGMENTAL EXPANSION COMPONENT, AND DELIVERY DEVICE AND VASCULAR IMPLANT SYSTEM COMPRISING SAME**

(30) Priority: 27.03.2023 CN 202310302571
(71) Applicant: AccuMedical Inc., Beijing 101300 (CN)
(72) Inventor: LU, Yiran, Beijing 101300 (CN); GAO, Hongliang, Beijing 101300 (CN); CHEN, Zhaohui, Beijing 101300 (CN)
(74) Representative: Gille Hrabal Partnerschaftsgesellschaft mbB Patentanwälte
(86) International application number: PCT/CN2023/122393
(87) International publication number: WO 2024/198292

(57) **Abstract**

The present application relates to a segmental expansion component, and a delivery device and vascular implant system comprising same. The segmental expansion component is provided with at least one segmental expansion unit, and the segmental expansion unit comprises an expandable portion, a proximal restraint portion connected to the proximal end of the expandable portion, and a distal restraint portion connected to the distal end of the expandable portion. The segmental expansion component is obtained by cross-weaving two or more wires into a mesh tube structure and performing segmental restraint according to a predetermined length. In the segmental expansion unit, when the expandable portion is in a radially expanded state, a ratio of the axial length of the expandable portion to the axial length of the proximal restraint portion is 8:1 to 20:1; and the expandable portion has 20 to 200 crossing points per inch of length. The segmental expansion component provided by the present application can obtain suitable distal axial thrust and radial support force without losing flexibility and pushability, thus ensuring the massaging and smoothing effect of the inner wall of an implant.

## Description

### TECHNICAL FIELD

The present application relates to the field of medical devices, and in particular, relates to a segmental expansion component, a delivery device and a vascular implant system including the same.

### BACKGROUND

Delivery of a self-expanding tubular implant (such as a stent) is generally achieved through a delivery system that delivers the self-expanding tubular implant from an extracorporeal proximal end (such as the radial artery or femoral artery) to the diseased blood vessel. The delivery system generally includes a pushing guidewire extending from the extracorporeal proximal end to the diseased blood vessel inside the body. An anti-unloading component (such as a silicone pad), an auxiliary pushing guidewire, and a radiopaque head are sequentially arranged starting from the distal end of the pushing guidewire. The auxiliary pushing guidewire is generally an extension of the pushing guidewire, and the anti-unloading component is generally arranged around the distal end of the pushing guidewire. However, the flexibility and pushability of the auxiliary pushing guidewire in this design still need further improvement.

CN214285319U discloses a bead-string-shaped component and a stent delivery system. The bead-string-shaped component includes an expandable body and at least two forming assemblies, and each of the forming assemblies includes a first component and a second component. The first component is provided with an inner cavity, and a partial segment of the expandable body is under restraint within the inner cavity to form a contraction segment of the bead-string-shaped component; alternatively, the first component is provided with an outer surface, and the inner surface of a partial segment of the expandable body is fixedly connected to the outer surface to form a contraction segment of the bead-string-shaped component. The second component is a tubular component, and the contraction segment is sleeved in the second component. An expansion segment of the bead-string-shaped component is formed between two adjacent contraction segments. The bead-string-shaped component facilitates the smooth release of the stent, improves the smoothness of stent delivery, and features a simple structure, ease of manufacturing, and high reliability.

The bead-string-shaped component provided in the prior art aims to provide a component that can facilitate the smooth release of the stent and make the stent fit with the diseased blood vessel. Meanwhile, due to the arrangement of the contraction segment, the contact area between the expandable body and the inner wall of other devices or the inner wall of the blood vessel is reduced, thereby reducing damage to the vascular intima. Additionally, the expansion segment of the expandable body deforms according to the direction and shape of the blood vessel, which is conducive to reducing the resistance of stent delivery and improving the smoothness of stent delivery.

In a conventional operation, after the self-expanding tubular implant is released, the tubular implant is flatteningd via a guidewire to flatten the inner wall of the tubular implant, further improving the fit between the tubular implant and the blood vessel. The bead-string-shaped component can directly flattening the inner wall of the tubular implant without being withdrawn from the body after the tubular implant is released. However, the process of massaging the inner wall is not examined in the bead-string-shaped component provided by the prior art. How to improve the flattening and wall-fitting effect on the inner wall at the distal end of the tubular implant while maintaining the flexibility of the distal end, as well as improving the safety of the distal end when massaging the inner wall of the tubular implant, is a direction that needs to be further explored.

Therefore, there is a need in the field to develop a delivery component for tubular implants and a delivery system including the same. The delivery component is provided with a segmental expansion component, with the expectation that the structure has better flexibility and pushability while maintaining better control over the distal end.

### SUMMARY

In view of the shortcomings of the prior art, a first object of the present application is to provide a segmental expansion component. The segmental expansion component is provided with at least one segmental expansion unit, and the segmental expansion unit includes one expandable part, a proximal restraint part connected to a proximal end of the expandable part, and a distal restraint part connected to a distal end of the expandable part; the segmental expansion component is obtained by cross-braiding two or more wires into a mesh-tubular structure and applying segmental restraints according to a predetermined length.

In the segmental expansion unit, when the expandable part is in a radially expanded state, a ratio of an axial length of the expandable part to an axial length of the proximal restraint part is 8:1 to 20:1 (e.g., 8.4:1, 8.8:1, 9.5:1, 10.2:1, 10.7:1, 11:1, 12:1, 13:1, 14:1, 15:1, 16:1, 17:1, 18:1, 19:1, etc.).

The expandable part has 20 to 200 crossing points per inch of length, for example, 25, 30, 35, 50, 70, 90, 120, 150, 170, 180, 190, etc., and preferably 50 to 120.

The segmental expansion component provided in the present application is integrally braided from wires and is braided according to a predetermined length ratio. By selecting the ratio of the axial length of the expandable part to the axial length of the proximal restraint part and limiting the braiding density (the number of crossing points per inch of length of the expandable part), it can be ensured that the segmental expansion component has appropriate flexibility and pushability during the pushing process, while also having an appropriate relatively small distal axial thrust and an appropriate relatively large radial support force after release. This may be because the longer the length of the expandable part is, the better the flexibility of the segmental expansion unit is, but the poorer the pushing force is. As a result, the radial support force of the segmental expansion unit is excessively small, and the massaging effect on the inner wall of the implant becomes worse. In addition, a longer length of the restraint part results in an excessively large distal axial thrust of the segmental expansion unit, which can easily cause vascular damage. In addition, the greater the number of crossing points per inch of length of the expandable part is, the higher the braiding density is. An appropriate braiding density also enables the segmental expansion component to have the appropriate radial support force and axial thrust.

The smaller the number of crossing points per inch of length of the expandable part in the expanded state is, the better the flexibility and the smaller the radial support force of the segmental expansion unit is. Therefore, an appropriate ratio (8:1 to 20:1) of the axial length of the appropriate expandable part to the axial length of the proximal restraint part, together with the number of crossing points per inch of length, enables the segmental expansion unit to have sufficient flexibility and pushability during the pushing process, while also obtaining sufficient radial support force and relatively small distal axial thrust after release, thereby achieving a better stent flattening effect.

In more cases, the smaller the wire diameter is, the larger the range of PPI can be selected. For example, when the wire diameter is 0.040 mm to 0.050 mm, the PPI can be selected in the range of 50 to 120, and in this case, the segmental expansion component demonstrates better pushing flexibility and pushability while having the appropriate distal axial thrust and radial support force.

The total length of the segmental expansion component described in the present application depends on the length of the tubular implant. Preferably, the segmental expansion component penetrates through the entire implant. Preferably, the total length of the segmental expansion component described in the present application is within the range of ≤ 70 mm. The segmental expansion component within the range of 70 mm can better satisfy the selection of the ratio of the axial length of the expandable part to the axial length of the proximal restraint part described above, as well as the influence of the braiding density on the radial support force and distal axial thrust.

Preferably, when the expandable part is in the expanded state, along a same axial cross-section, a projection point of a center point of the axial length of the expandable part onto an inner side of a cavity wall of the expandable part is point A, an intersection point between the proximal restraint part and the inner side of the cavity wall of the expandable part is point B, and an angle between a line connecting the point A and the point B and an axial direction is 10° to 30°, for example, 12°, 18°, 22°, 28°, etc.

Preferably, an intersection point between the distal restraint part of the expandable part and the inner side of the cavity wall of the expandable part is point B', and an angle between a line connecting the point A and the point B' and an axial direction is 10° to 30°, for example, 12°, 18°, 22°, 28°, etc.

Preferably, the angle between the line connecting the point A and the point B and the axial direction is the same as the angle between the line connecting the point A and the point B' and the axial direction. Generally, if a mesh tube is only restrained without performing directional differentiated heat setting, a solution will be obtained in which the angle between the line connecting the point A and the point B and the axial direction is the same as the angle between the line connecting the point A and the point B' and the axial direction.

If the angle between the line connecting the point A and the point B and the axial direction is excessively large, a high degree of expansion of the expandable part would be caused. As a result, the force conductivity of the segmental expansion component may be weakened, and the problem of bouncing or inability to achieve stable release of the tubular implant during the release process may be caused, thereby potentially creating safety risks during surgery. If the angle between the line connecting the point A and the point B and the axial direction is excessively small, a low degree of expansion of the expandable part would be caused. As a result, the flexibility of the segmental expansion component is weakened, and the contact area with the inner wall of the tubular implant is reduced, leading to an insignificant flattening effect on the inner wall of the tubular implant.

The setting of the dimensions of the expandable part and the restraint part of the segmental expansion component needs to match (be equal to, slightly smaller than, or smaller than) the inner diameter of the tubular implant. The absolute dimension of the segmental expansion component is not specifically limited in the present application, and may be selected by those skilled in the art based on actual needs. Exemplarily, when the segmental expansion component is used in neurovascular intervention surgery, preferably, the dimension of the inner diameter of the expandable part in the expanded state is 1.32 mm to 2.82 mm (e.g., 1.35 mm, 1.4 mm, 1.5 mm, 1.7 mm, 1.9 mm, 2.2 mm, 2.4 mm, 2.6 mm, 2.8 mm, etc.); the dimension of the inner diameter of the restraint part is ≤ 0.27 mm, for example, 0.26 mm, 0.23 mm, 0.20 mm, 0.16 mm, 0.14 mm, 0.12 mm, 0.05 mm, 0.03 mm, etc.

The material of the wires used for braiding is not specifically limited in the present application, provided that the mesh-tubular structure formed by braiding with the wires satisfies the foregoing limitation on the structure of the segmental expansion unit. The wires used for braiding may be exemplarily selected from Nitinol alloy materials or other alternative superelastic materials such as metals, alloys, or polymers, or shape-memory materials, or piezoelectric materials.

Preferably, the segmental expansion component is provided with two or more segmental expansion units, and the segmental expansion units are connected end to end; in adjacent segmental expansion units, a distal restraint part of a proximal segmental expansion unit and a proximal restraint part of a distal segmental expansion unit are a same restraint part.

It should be noted that the segmental expansion component described in the present application may be provided with only one segmental expansion unit, or may be provided with two or more segmental expansion units. When two or more segmental expansion units are present, the segmental expansion units are connected end to end, and in adjacent segmental expansion units, the distal restraint part of the proximal segmental expansion unit and the proximal restraint part of the distal segmental expansion unit are the same restraint part, that is, the connection way of the segmental expansion component (taking three segmental expansion units as an example) from proximal to distal may be exemplarily "the proximal restraint part of the first segmental expansion unit - the expandable part of the first segmental expansion unit - the proximal restraint part of the second segmental expansion unit - the expandable part of the second segmental expansion unit - the proximal restraint part of the third segmental expansion unit - the expandable part of the third segmental expansion unit".

Preferably, the segmental expansion unit is a mesh-tubular structure with both ends under restraint.

The mesh-tubular structure has certain expansibility, and an exemplary manufacturing method may be as follows: cross-braiding metal wires into a mesh tube, then allowing the mesh tube to present a certain expanded state, and setting. For example, with a cylindrical structure as a central axis, two or more metal wires are cross-braided around the central axis into a mesh-tubular structure. After heat setting is performed on the braided mesh tube at a setting temperature, the braided mesh tube is removed from the central axis to obtain the mesh-tubular structure. The setting temperature may be determined according to the material of the metal wire.

In a preferred embodiment, the segmental expansion component further includes a first force conduction auxiliary component; a distal end of the first force conduction auxiliary component is coaxially connected to and extends from a proximal end of a most proximal segmental expansion unit.

The first force conduction auxiliary component is 1 to 3 mesh-tubular expandable structures connected end to end with both ends under restraint, and a restraint part at a distal end of the first force conduction auxiliary component and a proximal restraint part of an adjacent segmental expansion unit are a same restraint part.

When the mesh-tubular expandable structure of the first force conduction auxiliary component is expanded, along a same axial cross-section, a projection point of a center point of an axial length of the mesh-tubular expandable structure onto a cavity wall of the mesh-tubular expandable structure is point C, an intersection point between a proximal restraint part of the mesh-tubular expandable structure and the mesh-tubular expandable structure is point D, and an angle between a line connecting the point C and the point D and an axial direction is a proximal transition angle of the mesh-tubular expandable structure of the first force conduction auxiliary component, the proximal transition angle of the mesh-tubular expandable structure being 0° to 10°.

Preferably, in the first force conduction auxiliary component, an intersection point between a distal restraint part of the mesh-tubular expandable structure and the mesh-tubular expandable structure is point D', and an angle between a line connecting the point C and the point D' and the axial direction is a distal transition angle of the mesh-tubular expandable structure of the first force conduction auxiliary component.

Preferably, in the mesh-tubular expandable structure of the first force conduction auxiliary component, the proximal transition angle and the distal transition angle are the same. Generally, if a mesh tube is only restrained without performing directional differentiated heat setting, a solution will be obtained in which the proximal transition angle and the distal transition angle are the same.

The function of the first force conduction auxiliary component is to improve the control of the proximal end (especially the extracorporeal proximal end) over the segmental expansion component without reducing the outer diameter and radial support force of the segmental expansion component. This may be because the proximal transition angle (and the distal transition angle) of the first force conduction auxiliary component is smaller, and when the proximal end rotates, tilts, pushes, or performs other operations, the force is more easily transmitted to the expandable part and the distal end.

Preferably, in the first force conduction auxiliary component, an angle of the proximal transition angle of the mesh-tubular expandable structure gradually increases from proximal to distal.

The size of the proximal transition angle of the mesh-tubular expandable structure of the first force conduction auxiliary component can be achieved by adjusting the length of the expandable part. Generally, the angle of the transition angle is reduced by shortening the length of the expandable part. In addition, in the case where the length remains unchanged, the angle of the transition angle can also be reduced by pre-setting.

Preferably, the segmental expansion component further includes a second force conduction auxiliary component.

A proximal end of the second force conduction auxiliary component is coaxially connected to and extends from a distal end of the most distal segmental expansion unit.

The second force conduction auxiliary component is 1 to 3 mesh-tubular expandable structures connected end to end with both ends under restraint, and a restraint part at a distal end of the second force conduction auxiliary component and a distal restraint part of an adjacent segmental expansion unit are a same restraint part.

When the mesh-tubular expandable structure of the second force conduction auxiliary component is expanded, along a same axial cross-section, a projection point of a center point of an axial length of the mesh-tubular expandable structure onto a cavity wall of the mesh-tubular expandable structure is point E, an intersection point between a proximal restraint part of the mesh-tubular expandable structure and the mesh-tubular expandable structure is point F, and an angle between a line connecting the point E and the point F and an axial direction is a proximal transition angle of the mesh-tubular expandable structure of the second force conduction auxiliary component, the proximal transition angle of the mesh-tubular expandable structure being 0° to 10°.

Preferably, in the second force conduction auxiliary component, an intersection point between a distal restraint part of the mesh-tubular expandable structure and the mesh-tubular expandable structure is point F', and an angle between a line connecting the point E and the point F' and the axial direction is a distal transition angle of the mesh-tubular expandable structure of the second force conduction auxiliary component.

Preferably, in the mesh-tubular expandable structure of the second force conduction auxiliary component, the proximal transition angle and the distal transition angle are the same.

The function of the second force conduction auxiliary component is to more easily control the axial force at the distal end within an appropriate range. When the force is conducted to the expandable part, the force is dispersed due to the relatively large expansion diameter, and the axial force is excessively small. The second force conduction auxiliary component re-aggregates the dispersed force, such that the axial force can be controlled within an appropriate range. In addition, the distal end of the tubular implant may be arranged at the expandable structure of the second force conduction auxiliary component, and the relatively small transition angle of the second force conduction auxiliary component enables the distal end of the tubular implant to be stably released without causing the problem of bouncing of the distal end of the implant during the release process.

Preferably, in the second force conduction auxiliary component, the transition angle of the mesh-tubular expandable structure gradually decreases from proximal to distal.

The size of the proximal transition angle of the mesh-tubular expandable structure of the second force conduction auxiliary component can be achieved by adjusting the length of the expandable part. Generally, the angle of the transition angle is reduced by shortening the length of the expandable part. In addition, in the case where the length remains unchanged, the angle of the transition angle can also be reduced by a preforming method.

Preferably, lengths of the restraint parts are the same.

The lengths of the restraint parts are the same, making structural adjustments to the segmental expansion component easier.

Preferably, methods of the segmental restraints include any one or a combination of at least two of pre-setting, bonding or welding or hot-melt connecting to a substrate, directly bonding or welding or hot-melt connecting extended wires, or sleeving a restraint body on an outer side.

The methods of the segmental restraints are not specifically limited in the present application, and any restraint method capable of forming a neck part can be used in the present application.

In addition, to enable the segmental expansion component to be visible inside the body, the restraint part may be selectively made of a radiopaque material, and the radiopaque material includes but is not limited to platinum, tantalum, palladium, or other radiopaque materials.

A second object of the present application is to provide a vascular implant delivery device. The vascular implant delivery device includes:
a pushing guidewire;
the segmental expansion component described in the first object, connected to a distal end of the pushing guidewire;
a radiopaque marker tip connected to a distal end of the segmental expansion component; and
a vascular implant placement and retractio assisting component fixedly sleeved on the distal end of the pushing guidewire, where the vascular implant placement and withdrawl assisting component includes a fixing part configured to be fixedly sleeved on the distal end of the pushing guidewire and an opening part arranged at a distal end of the fixing part, the opening part having a structure gradually enlarging from a proximal end to a distal end.

Preferably, the opening part includes any one of a trumpet-shaped opening with an opening facing the distal end and a splint radially presented from the proximal end to the distal end.

Preferably, the radiopaque marker tip includes a core part obtained by extension of the segmental expansion component, and a radiopaque wire wound and fixed on an outer side of the core part. A helical coil is an optional structure for the radiopaque wire, and those skilled in the art may select a structure with equivalent flexibility, toughness, and strength as a substitute. Typically, but not limited, the helical coil of the radiopaque marker tip may be formed by a single wire or a plurality of wires.

The radiopaque metal material of the radiopaque marker tip is a radiopaque material, which includes but is not limited to platinum, tantalum, palladium, or other radiopaque materials.

Preferably, the vascular implant delivery device further includes an annular anti-disengagement part sleeved on the proximal end of the segmental expansion component, where the annular anti-disengagement part has radial compressive elasticity.

A third object of the present application is to provide a vascular implant system. The vascular implant system includes:
the vascular implant delivery device described in the second object; and
a vascular implant sleeved on an outer side of the segmental expansion component of the vascular implant delivery device.

Alternatively, the vascular implant system includes:
a pushing guidewire;
the segmental expansion component described in the first object, connected to a distal end of the pushing guidewire;
a radiopaque marker tip connected to a distal end of the segmental expansion component; and
a vascular implant placement and withdrawl assisting component fixedly sleeved on the distal end of the pushing guidewire, where the vascular implant placement and withdrawl assisting component includes a fixing part configured to be fixedly sleeved on the distal end of the pushing guidewire and an opening part arranged at a distal end of the fixing part, the opening part having a structure gradually enlarging from a proximal end to a distal end; and
a vascular implant sleeved on an outer side of the segmental expansion component of the vascular implant delivery device.

Preferably, the vascular implant system further includes a sheath, configured to accommodate the vascular implant delivery device with the vascular implant sleeved thereon.

Compared with the prior art, the present application has the following beneficial effects:
For the segmental expansion component provided in the present application, by limiting the segmental structure and the expandable part PPI, the segmental expansion component obtains appropriate distal axial thrust and radial support force without losing flexibility and pushability, thereby ensuring the massaging and flattening effect on the inner wall of the implant. After the self-expanding stent is fully released, the arrangement of the segmental expansion component provided in the present application (including the ratio of the axial length of the expandable part to the axial length of the proximal restraint part and the PPI) can effectively flattening the self-expanding stent, and can transmit the operation of the (extracorporeal) proximal end on the pushing guidewire to the segmental expansion component. Effective contact with the inner wall (particularly the distal inner wall) of the self-expanding stent is enabled without excessively extending the distal end of the self-expanding stent, and there is a certain radial force to flatten the inner wall of the stent and improve wall-fitting. In addition, the relatively small distal axial thrust can maintain the flexibility of the distal end and improve the safety of the distal end.

### BRIEF DESCRIPTION OF THE DRAWINGS

FIG. 1 is a schematic structural diagram of the segmental expansion component according to Example 1;
FIG. 2 is a schematic structural diagram of a first expandable part 100; and
FIG. 3 is a schematic structural diagram of the segmental expansion component according to Example 10.

### DETAILED DESCRIPTION

The following further explains the technical solutions of the present application with reference to specific embodiments. However, it should be noted that the specific embodiments are merely a specific implementation and explanation of the essence of the technical solutions of the present application, and should not be construed as a limitation to the protection scope of the present application.

The present application will be further described in detail with reference to the accompanying drawings and examples. It can be understood that the specific examples described herein are merely used to explain the related application, but are not intended to limit the application. In addition, it should be noted that, for ease of description, only parts related to the relevant application are shown in the accompanying drawings.

In the description of the present application, it should be noted that, unless explicitly specified and limited otherwise, terms such as "mounted", "connected", and "coupled" should be broadly interpreted. For example, they could denote fixed connections, detachable connections, or integral connections; they could represent mechanical connections or electrical connections; and they could mean direct connections or indirect connections through intermediation. For those of ordinary skill in the art, the specific meanings of the terms described above in the present application can be understood according to the specific circumstances.

In the description of the present application, it should be understood that the terms "distal end" and "proximal end" herein should be understood as viewed from the direction of the surgical operator. The "distal end" is an end distal to the surgical operator, and the "proximal end" is an end proximal to the surgical operator. The term "axial direction" herein should be understood as a direction in which a stent is pushed or a length direction of a guidewire, and "radial direction" should be understood as a direction perpendicular to the "axial direction".

In the description of the present application, it should be noted that the examples in the present application and the features in the examples may be combined with each other in the absence of conflicts.

### Example 1

For a segmental expansion component, 18 Nitinol alloy wires with a diameter of 0.046 mm were used as braiding wires. The braiding wires were braided on a mandrel with a diameter of 3.5 mm according to a PPI of 85. The braiding method involved a two-over-two-under braiding method (specifically, one warp and four adjacent wefts crossing over the warp were a set of braiding wires, and the set of braiding wires included four braiding points, which were two positive braiding points and two negative braiding points, respectively, where the two positive braiding points were adjacent or the two negative braiding points were adjacent). After being maintained at 520 °C for 5 minutes for heat setting, the mandrel was removed to obtain a mesh-tubular structure with a length of 60 mm.

The mesh-tubular structure (in a natural state) was segmented into four segments every 8 mm, and restraints were applied at the segmented points. The restraint method involved welding and arranging a platinum alloy cylinder (a radiopaque component) with an outer diameter of 0.41 mm and a length of 0.5 mm inside the proximal starting part of the segment, obtaining the segmental expansion component (the ratio of the axial length of the expandable part to the axial length of the proximal restraint part being 15:1). The dimension of the outer diameter of the expandable part after expansion was 2.5 mm, and the angle between the line connecting point A and point B and the axial direction was 15.58°.

### Example 2

The difference from Example 1 was only that the segment ratio of the mesh-tubular structure was changed to five segments every 6 mm (the ratio of the axial length of the expandable part to the axial length of the proximal restraint part being 11:1).

The dimension of the outer diameter of the expandable part of the segmental expansion component obtained in Example 2 after expansion was 2.3 mm. Moreover, the angle between the line connecting point A and point B and the axial direction was 18.97°.

### Example 3

The difference from Example 1 was only that the segment ratio of the mesh-tubular structure was changed to three segments every 10.5 mm (the ratio of the axial length of the expandable part to the axial length of the proximal restraint part being 20:1).

The dimension of the outer diameter of the expandable part of the segmental expansion component obtained in Example 3 after expansion was 2.7 mm. Moreover, the angle between the line connecting point A and point B and the axial direction was 12.90°.

### Examples 4 to 6

The difference from Example 1 was only that the braiding density PPI of the mesh-tubular structure was changed to 50 (Example 4), 120 (Example 5), and 200 (Example 6).

### Example 7

For a segmental expansion component, 18 Nitinol alloy wires with a diameter of 0.046 mm were used as braiding wires. The braiding wires were braided on a mandrel with a diameter of 3.5 mm according to a PPI of 85. The braiding method involved a two-over-two-under braiding method (specifically, one warp and four adjacent wefts crossing over the warp were a set of braiding wires, and the set of braiding wires included four braiding points, which were two positive braiding points and two negative braiding points, respectively, where the two positive braiding points were adjacent or the two negative braiding points were adjacent). After being maintained at 520 °C for 5 minutes for heat setting, the mandrel was removed to obtain a mesh-tubular structure with a length of 80 mm.

The mesh-tubular structure (in a natural state) was segmented into six segments every 8 mm, and restraints were applied at the segmented points. The restraint method involved welding and arranging a platinum alloy cylinder with an outer diameter of 0.41 mm and a length of 0.5 mm inside the proximal starting part of the segment, obtaining the segmental expansion component (the ratio of the axial length of the expandable part to the axial length of the proximal restraint part being 15:1). The dimension of the outer diameter of the expandable part after expansion was 2.5 mm, and the angle between the line connecting point A and point B and the axial direction was 15.58°.

### Example 8

The difference from Example 1 was that for a segmental expansion component, 16 Nitinol alloy wires with a diameter of 0.09 mm were used as braiding wires. The braiding wires were braided on a mandrel with a diameter of 4.5 mm according to a PPI of 30. The braiding method involved a two-over-two-under braiding method (specifically, one warp and four adjacent wefts crossing over the warp were a set of braiding wires, and the set of braiding wires included four braiding points, which were two positive braiding points and two negative braiding points, respectively, where the two positive braiding points were adjacent or the two negative braiding points were adjacent). After being maintained at 530 °C for 6 minutes for heat setting, the mandrel was removed to obtain a mesh-tubular structure with a length of 80 mm.

The mesh-tubular structure (in a natural state) was segmented into four segments every 12 mm, and restraints were applied at the segmented points. The restraint method involved welding and arranging a platinum alloy cylinder with an outer diameter of 0.5 mm and a length of 0.6 mm inside the proximal starting part of the segment, obtaining the segmental expansion component (the ratio of the axial length of the expandable part to the axial length of the proximal restraint part being 19:1). The dimension of the outer diameter of the expandable part after expansion was 3.7 mm, and the angle between the line connecting point A and point B and the axial direction was 15.69°.

### Example 9

The difference from Example 1 was that for a segmental expansion component, 16 Nitinol alloy wires with a diameter of 0.09 mm were used as braiding wires. The braiding wires were braided on a mandrel with a diameter of 4.5 mm according to a PPI of 30. The braiding method involved a two-over-two-under braiding method (specifically, one warp and four adjacent wefts crossing over the warp were a set of braiding wires, and the set of braiding wires included four braiding points, which were two positive braiding points and two negative braiding points, respectively, where the two positive braiding points were adjacent or the two negative braiding points were adjacent). After being maintained at 530 °C for 6 minutes for heat setting, the mandrel was removed to obtain a mesh-tubular structure with a length of 80 mm.

The mesh-tubular structure (in a natural state) was segmented into nine segments every 5.5 mm, and restraints were applied at the segmented points. The restraint method involved welding and arranging a platinum alloy cylinder with an outer diameter of 0.5 mm and a length of 0.6 mm inside the proximal starting part of the segment, obtaining the segmental expansion component (the ratio of the axial length of the expandable part to the axial length of the proximal restraint part being 8:1). The dimension of the outer diameter of the expandable part after expansion was 3.5 mm, and the angle between the line connecting point A and point B and the axial direction was 28.62°.

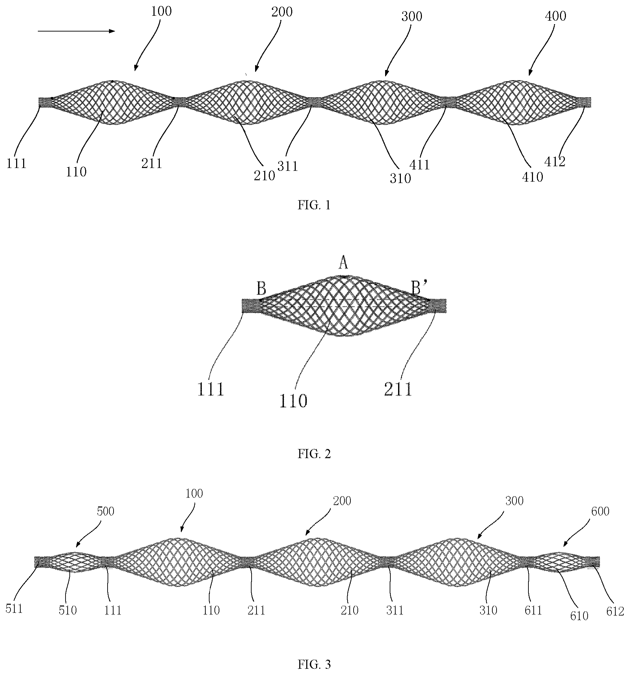

Examples 1 to 9 have similar structures. Taking Example 1 as an example, a specific schematic structural diagram of the obtained segmental expansion component is shown in FIG. 1 (FIG. 1 being a schematic structural diagram of the segmental expansion component according to Example 1), and the segmental expansion component is provided with four segmental expansion units. A first segmental expansion unit 100 includes a first expandable part 110 and a first proximal restraint part 111 connected to the proximal end of the first expandable part 110; a second segmental expansion unit 200 includes a second expandable part 210 and a second proximal restraint part 211 connected to the proximal end of the second expandable part 210; a third segmental expansion unit 300 includes a third expandable part 310 and a third proximal restraint part 311 connected to the proximal end of the third expandable part 310; and a fourth segmental expansion unit 400 includes a fourth expandable part 410 and a fourth proximal restraint part 411 connected to the proximal end of the fourth expandable part 410. The distal restraint part of the fourth segmental expansion unit 400 is the first distal restraint part 412.

In addition, the distal restraint part of the first segmental expansion unit 100 is the second proximal restraint part 211, the distal restraint part of the second segmental expansion unit 200 is the third proximal restraint part 311, and the distal restraint part of the third segmental expansion unit 300 is the fourth proximal restraint part 411.

In FIG. 1, the arrow direction points from the proximal end to the distal end.

As shown in FIG. 2 (FIG. 2 being a schematic structural diagram of the first expandable part 100), taking the first expandable part 100 as an example, in the expanded state thereof, along the same axial cross-section, the projection point of the center point of the axial length of the expandable part 100 onto the inner side of the cavity wall of the expandable part is point A, the intersection point between the proximal restraint part and the inner side of the cavity wall of the expandable part is point B, and the intersection point between the distal restraint part and the inner side of the cavity wall of the expandable part is point B'.

### Example 10

For a segmental expansion component, 18 Nitinol alloy wires with a diameter of 0.046 mm were used as braiding metal wires. The braiding metal wires were braided on a mandrel with a diameter of 3.5 mm according to a PPI of 85. The braiding method involved a two-over-two-under braiding method (specifically, one warp and four adjacent wefts crossing over the warp were a set of braiding wires, and the set of braiding wires included four braiding points, which were two positive braiding points and two negative braiding points, respectively, where the two positive braiding points were adjacent or the two negative braiding points were adjacent). After being maintained at 520 °C for 5 minutes for heat setting, the mandrel was removed to obtain a mesh-tubular structure with a length of 60 mm.

The mesh-tubular structure (in a natural state) was segmented according to a first segment of 4 mm, a second segment of 8 mm, a third segment of 8 mm, a fourth segment of 8 mm, and a fifth segment of 4 mm, and restraints were applied at the segmented points. The restraint method involved welding and arranging a platinum alloy cylinder with an outer diameter of 0.41 mm and a length of 0.5 mm inside the proximal starting part of the segment, obtaining the segmental expansion component. The first segment and the fifth segment had an outer diameter of 0.9 mm, and the second segment, the third segment, and the fourth segment had an outer diameter of 2.5 mm. In addition, the transition angles of the first segment and the fifth segment were 7.97°, and the angles between the line connecting point A and point B of the second segment, the third segment, and the fourth segment and the axial direction were 15.58°.

A specific schematic structural diagram of the segmental expansion component obtained in Example 10 is shown in FIG. 3 (FIG. 3 being a schematic structural diagram of the segmental expansion component according to Example 10), and the segmental expansion component is provided with five segments, which are, in sequence from proximal to distal, a first force conduction auxiliary component 500 including a first mesh-tubular expandable structure 510 and a fifth proximal restraint part 511 connected to the proximal end of the mesh-tubular expandable structure 510; a first segmental expansion unit 100 including a first expandable part 110 and a first proximal restraint part 111 connected to the proximal end of the first expandable part 110; a second segmental expansion unit 200 including a second expandable part 210 and a second proximal restraint part 211 connected to the proximal end of the second expandable part 210; a third segmental expansion unit 300 including a third expandable part 310 and a third proximal restraint part 311 connected to the proximal end of the third expandable part 310; and a second force conduction auxiliary component 600 including a second mesh-tubular expandable structure 610 and a sixth proximal restraint part 611 connected to the proximal end of the mesh-tubular expandable structure 610. The distal restraint part of the second force conduction auxiliary component 600 is the second distal restraint part 612.

In addition, the distal restraint part of the first mesh-tubular expandable structure 510 is the first proximal restraint part 111, the distal restraint part of the first segmental expansion unit 100 is the second proximal restraint part 211, the distal restraint part of the second segmental expansion unit 200 is the third proximal restraint part 311, and the distal restraint part of the third segmental expansion unit 300 is the sixth proximal restraint part 611.

In FIG. 3, the arrow direction points from the proximal end to the distal end.

### Example 11

For a segmental expansion component, 18 Nitinol alloy wires with a diameter of 0.046 mm were used as braiding metal wires. The braiding metal wires were braided on a mandrel with a diameter of 3.5 mm according to a PPI of 85. The braiding method involved a two-over-two-under braiding method (specifically, one warp and four adjacent wefts crossing over the warp were a set of braiding wires, and the set of braiding wires included four braiding points, which were two positive braiding points and two negative braiding points, respectively, where the two positive braiding points were adjacent or the two negative braiding points were adjacent). After being maintained at 520 °C for 5 minutes for heat setting, the mandrel was removed to obtain a mesh-tubular structure with a length of 60 mm.

The mesh-tubular structure (in a natural state) was segmented according to a first segment of 1 mm, a second segment of 3 mm, a third segment of 8 mm, a fourth segment of 8 mm, a fifth segment of 8 mm, and a sixth segment of 4 mm, and restraints were applied at the segmented points. The restraint method involved welding and arranging a platinum alloy cylinder with an outer diameter of 0.41 mm and a length of 0.5 mm inside the proximal starting part of the segment, obtaining the segmental expansion component. The first segment had an outer diameter of 0.42 mm, the second segment had an outer diameter of 0.6 mm, the third segment, the fourth segment, and the fifth segment had an outer diameter of 2.5 mm, and the sixth segment had an outer diameter of 0.9 mm. In addition, the transition angle of the first segment was 1.14°, and the transition angle of the second segment was 4.35°; the angles between the line connecting point A and point B of the third segment, the fourth segment, and the fifth segment and the axial direction were still 15.58°, and the transition angle of the sixth segment was 7.97°.

### Example 12

For a segmental expansion component, 18 Nitinol alloy wires with a diameter of 0.046 mm were used as braiding metal wires. The braiding metal wires were braided on a mandrel with a diameter of 6 mm according to a PPI of 85. The braiding method involved a two-over-two-under braiding method (specifically, one warp and four adjacent wefts crossing over the warp were a set of braiding wires, and the set of braiding wires included four braiding points, which were two positive braiding points and two negative braiding points, respectively, where the two positive braiding points were adjacent or the two negative braiding points were adjacent). After being maintained at 520 °C for 5 minutes for heat setting, the mandrel was removed to obtain a mesh-tubular structure with a length of 60 mm.

The mesh-tubular structure (in a natural state) was segmented into four segments every 8 mm, and restraints were applied at the segmented points. The restraint method involved welding and arranging a platinum alloy cylinder with an outer diameter of 0.41 mm and a length of 0.5 mm inside the proximal starting part of the segment, obtaining the segmental expansion component (the ratio of the axial length of the expandable part to the axial length of the proximal restraint part being 15:1). The dimension of the outer diameter of the expandable part after expansion was 5.3 mm, and the angle between the line connecting point A and point B and the axial direction was 33.12°.

### Comparative Example 1

The difference from Example 1 was only that the braiding density PPI of the mesh-tubular structure was changed to 15.

### Comparative Example 2

For a segmental expansion component, 18 Nitinol alloy wires with a diameter of 0.046 mm were used as braiding metal wires. The braiding metal wires were braided on a mandrel with a diameter of 3.5 mm according to a PPI of 85. The braiding method involved a two-over-two-under braiding method (specifically, one warp and four adjacent wefts crossing over the warp were a set of braiding wires, and the set of braiding wires included four braiding points, which were two positive braiding points and two negative braiding points, respectively, where the two positive braiding points were adjacent or the two negative braiding points were adjacent). After being maintained at 520 °C for 5 minutes for heat setting, the mandrel was removed to obtain a mesh-tubular structure with a length of 60 mm.

The mesh-tubular structure (in a natural state) was segmented into ten segments every 3 mm, and restraints were applied at the segmented points. The restraint method involved welding and arranging a platinum alloy cylinder with an outer diameter of 0.41 mm and a length of 0.5 mm inside the proximal starting part of the segment, obtaining the segmental expansion component (the ratio of the axial length of the expandable part to the axial length of the proximal restraint part being 5:1). The dimension of the outer diameter of the expandable part after expansion was 0.6 mm, and the angle between the line connecting point A and point B and the axial direction was 4.35°.

### Comparative Example 3

For a segmental expansion component, 18 Nitinol alloy wires with a diameter of 0.046 mm were used as braiding metal wires. The braiding metal wires were braided on a mandrel with a diameter of 3.5 mm according to a PPI of 85. The braiding method involved a two-over-two-under braiding method (specifically, one warp and four adjacent wefts crossing over the warp were a set of braiding wires, and the set of braiding wires included four braiding points, which were two positive braiding points and two negative braiding points, respectively, where the two positive braiding points were adjacent or the two negative braiding points were adjacent). After being maintained at 520 °C for 5 minutes for heat setting, the mandrel was removed to obtain a mesh-tubular structure with a length of 60 mm.

The mesh-tubular structure (in a natural state) was segmented into two segments every 12.5 mm, and restraints were applied at the segmented points. The restraint method involved welding and arranging a platinum alloy cylinder with an outer diameter of 0.41 mm and a length of 0.5 mm inside the proximal starting part of the segment, obtaining the segmental expansion component (the ratio of the axial length of the expandable part to the axial length of the proximal restraint part being 24:1). The dimension of the outer diameter of the expandable part after expansion was 0.6 mm, and the angle between the line connecting point A and point B and the axial direction was 11.73°.

### Performance test:

The proximal ends of the segmental expansion components of the examples and the comparative examples were fixedly connected to the distal end of a 100 cm pushing guidewire to obtain test samples, which were used for the following performance tests.
(1) Trackability: The test sample was pushed into a trackability test fixture, and whether the test sample could smoothly pass through bends was recorded. The trackability test fixture includes a coplanar entry channel and a vascular model with a curved path, and the curved path includes four semicircular alternating curves with a radius of curvature of 2 mm.
(2) Distal force value: The test sample was placed in a straight vascular model, and a 200 mN force was applied to the proximal end. The distal force value was monitored using a full-module push-pull force gauge.
(3) Radial support force: A radial support force tester (MSI RS550) was used at a temperature of 37±2 °C. The initial diameter of the fixture was set to be 5.5 mm, and then the expandable part of the segmental expansion component was placed in the fixture. Subsequently, the diameter of the fixture was gradually reduced to 0.4 mm at a rate of 0.1 mm/s, and then gradually expanded to 5.5 mm at a rate of 0.1 mm/s. The maximum force value during the process of change was recorded.

The test results are shown in Table 1.

**Table 1 Performance test results**

| Example | Smoothly passed through bends or not | Distal force value (mN) | Radial support force (N) |
|---|---|---|---|
| Example 1 | Smoothly passed through bends | 4 | 0.53 |
| Example 2 | Smoothly passed through bends | 5 | 0.51 |
| Example 3 | Smoothly passed through bends | 3 | 0.55 |
| Example 4 | Smoothly passed through bends | 3 | 0.48 |
| Example 5 | Smoothly passed through bends | 5 | 0.79 |
| Example 6 | Smoothly passed through bends | 6 | 1.04 |
| Example 7 | Smoothly passed through bends | 3 | 0.53 |
| Example 8 | Smoothly passed through bends | 5 | 0.66 |
| Example 9 | Smoothly passed through bends | 6 | 0.63 |
| Example 10 | Smoothly passed through bends | 4 | 0.53 |
| Example 11 | Smoothly passed through bends | 5 | 0.53 |
| Example 12 | Smoothly passed through bends | 3 | 0.58 |
| Comparative Example 1 | Failed to pass through bends | 1 | 0.23 |
| Comparative Example 2 | Failed to pass through bends | 10 | 0.15 |
| Comparative Example 3 | Failed to pass through bends | 1 | 0.56 |

It can be seen from Table 1 that the segmental expansion components provided in the present application can all smoothly pass through the predetermined bends formed by four semicircular alternating curves with a radius of curvature of 2 mm. In addition, a 200 mN pushing force is applied to the proximal end, and the distal end can maintain a relatively small distal axial thrust of 3 mN to 6 mN and maintain an appropriate radial support force.

In Table 1, for Comparative Example 1, because the number of crossing points per inch of length of the expandable part is excessively small, and the axial thrust and radial support force are excessively small, the test sample fails to pass through the vascular model with a curved path. For Comparative Example 2, because the ratio of the axial length of the expandable part to the axial length of the proximal restraint part is excessively small, the flexibility becomes poor, and the test sample fails to pass through the vascular model with a curved path. For Comparative Example 3, because the ratio of the axial length of the expandable part to the axial length of the proximal restraint part is excessively large, and the axial thrust is excessively small, the test sample fails to pass through the vascular model with a curved path.

Finally, it should be noted that the examples described above are merely intended for illustrating the technical solutions of the present application, and not for limiting the present application. Although the present application is described in detail with reference to the foregoing examples, those of ordinary skill in the art should understand that they may still make modifications to the technical solutions described in the foregoing examples or make equivalent replacements to some or all technical features thereof. These modifications or replacements do not cause the essence of the corresponding technical solutions to deviate from the scope of the technical solutions of the examples of the present application.

## Claims

1. A segmental expansion component, wherein the segmental expansion component is provided with at least one segmental expansion unit, and the segmental expansion unit comprises one expandable part, a proximal restraint part connected to a proximal end of the expandable part, and a distal restraint part connected to a distal end of the expandable part; the segmental expansion component is obtained by cross-braiding two or more wires into a mesh-tubular structure and applying segmental restraints according to a predetermined length;
in the segmental expansion unit, when the expandable part is in a radially expanded state, a ratio of an axial length of the expandable part to an axial length of the proximal restraint part is 8:1 to 20:1; and
the expandable part has 20 to 200 crossing points per inch of length.

2. The segmental expansion component as claimed in claim 1, wherein when the expandable part is in the expanded state, along a same axial cross-section, a projection point of a center point of the axial length of the expandable part onto an inner side of a cavity wall of the expandable part is point A, an intersection point between the proximal restraint part and the inner side of the cavity wall of the expandable part is point B, and an angle between a line connecting the point A and the point B and an axial direction is 10° to 30°.

3. The segmental expansion component as claimed in claim 1, wherein the segmental expansion component is provided with two or more segmental expansion units, and the segmental expansion units are connected end to end; in adjacent segmental expansion units, a distal restraint part of a proximal segmental expansion unit and a proximal restraint part of a distal segmental expansion unit are a same restraint part.

4. The segmental expansion component as claimed in claim 1, wherein the segmental expansion component further comprises a first force conduction auxiliary component;
a distal end of the first force conduction auxiliary component is coaxially connected to and extends from a proximal end of a most proximal segmental expansion unit;
the first force conduction auxiliary component is 1 to 3 mesh-tubular expandable structures connected end to end with both ends under restraint, and a restraint part at a distal end of the first force conduction auxiliary component and a proximal restraint part of an adjacent segmental expansion unit are a same restraint part; and
when the mesh-tubular expandable structure of the first force conduction auxiliary component is expanded, along a same axial cross-section, a projection point of a center point of an axial length of the mesh-tubular expandable structure onto a cavity wall of the mesh-tubular expandable structure is point C, an intersection point between a proximal restraint part of the mesh-tubular expandable structure and the mesh-tubular expandable structure is point D, and an angle between a line connecting the point C and the point D and an axial direction is a proximal transition angle of the mesh-tubular expandable structure of the first force conduction auxiliary component, the proximal transition angle of the mesh-tubular expandable structure being 0° to 10°.

5. The segmental expansion component as claimed in claim 4, wherein in the first force conduction auxiliary component, an angle of the proximal transition angle of the mesh-tubular expandable structure gradually increases from proximal to distal.

6. The segmental expansion component as claimed in claim 1, wherein the segmental expansion component further comprises a second force conduction auxiliary component;
a proximal end of the second force conduction auxiliary component is coaxially connected to and extends from a distal end of a most distal segmental expansion unit;
the second force conduction auxiliary component is 1 to 3 mesh-tubular expandable structures connected end to end with both ends under restraint, and a restraint part at a distal end of the second force conduction auxiliary component and a distal restraint part of an adjacent segmental expansion unit are a same restraint part; and
when the mesh-tubular expandable structure of the second force conduction auxiliary component is expanded, along a same axial cross-section, a projection point of a center point of an axial length of the mesh-tubular expandable structure onto a cavity wall of the mesh-tubular expandable structure is point E, an intersection point between a proximal restraint part of the mesh-tubular expandable structure and the mesh-tubular expandable structure is point F, and an angle between a line connecting the point E and the point F and an axial direction is a proximal transition angle of the mesh-tubular expandable structure of the second force conduction auxiliary component, the proximal transition angle of the mesh-tubular expandable structure being 0° to 10°.

7. The segmental expansion component as claimed in claim 6, wherein in the second force conduction auxiliary component, the transition angle of the mesh-tubular expandable structure gradually decreases from proximal to distal.

8. The segmental expansion component as claimed in claim 1, wherein lengths of the restraint parts are the same.

9. The segmental expansion component as claimed in claim 1, wherein methods of the segmental restraints comprise any one or a combination of at least two of pre-setting, bonding or welding or hot-melt connecting to a substrate, directly bonding or welding or hot-melt connecting extended wires, or sleeving a restraint body on an outer side.

10. A vascular implant delivery device, comprising:
a pushing guidewire;
the segmental expansion component as claimed in any one of claims 1 to 9, connected to a distal end of the pushing guidewire;
a radiopaque marker tip connected to a distal end of the segmental expansion component; and
a vascular implant placement and withdrawl assisting component fixedly sleeved on the distal end of the pushing guidewire, wherein the vascular implant placement and withdrawl assisting component comprises a fixing part configured to be fixedly sleeved on the distal end of the pushing guidewire and an opening part arranged at a distal end of the fixing part, the opening part having a structure gradually enlarging from a proximal end to a distal end.

11. A vascular implant system, comprising:
the vascular implant delivery device as claimed in claim 10; and
a vascular implant sleeved on an outer side of the segmental expansion component of the vascular implant delivery device;
or,
the vascular implant system comprises:
a pushing guidewire;
the segmental expansion component as claimed in any one of claims 1 to 9, connected to a distal end of the pushing guidewire;
a radiopaque marker tip connected to a distal end of the segmental expansion component;
a vascular implant placement and withdrawl assisting component fixedly sleeved on the distal end of the pushing guidewire, wherein the vascular implant placement and withdrawl assisting component comprises a fixing part configured to be fixedly sleeved on the distal end of the pushing guidewire and an opening part arranged at a distal end of the fixing part, the opening part having a structure gradually enlarging from a proximal end to a distal end; and
a vascular implant sleeved on an outer side of the segmental expansion component of the vascular implant delivery device.

12. The vascular implant system as claimed in claim 11, wherein the vascular implant system further comprises a sheath, configured to accommodate the vascular implant delivery device with the vascular implant sleeved thereon.
